# EUROPEAN PATENT SPECIFICATION

(11) **EP 2 330 420 B1**
(45) Date of publication and mention of the grant of the patent: **06.09.2017**
(21) Application number: 09809743.9
(22) Date of filing: 31.07.2009
(51) Int. Cl.: G01N 33/564, G01N 33/68, G01N 33/53

(54) **DIAGNOSIS METHOD AND DIAGNOSIS KIT FOR CLINICAL AMYOPATHIC DERMATOMYOSITIS**
DIAGNOSEVERFAHREN UND DIAGNOSEKIT FÜR KLINISCHE AMYOPATHISCHE DERMATOMYOSITIS
PROCÉDÉ DE DIAGNOSTIC ET KIT DE DIAGNOSTIC POUR LA DERMATOMYOSITE AMYOPATHIQUE CLINIQUE

(30) Priority: 01.09.2008 JP 2008223789
(43) Date of publication of application: 08.06.2011
(73) Proprietor: Keio University, Tokyo 108-8345 (JP)
(72) Inventor: KUWANA, Masataka, Tokyo 160-8582 (JP); SATO, Shinji, Tokyo 160-8582 (JP); FUJITA, Takashi, Kyoto-shi Kyoto 606-8507 (JP)
(74) Representative: Müller-Boré & Partner Patentanwälte PartG mbB
(86) International application number: PCT/JP2009/063633
(87) International publication number: WO 2010/024089

(56) References cited:
- WO-A2-2005/016962
- US-A- 6 160 107
- SHINJI SATO ET AL: "RNA helicase encoded by melanoma differentiation-associated gene 5 is a major autoantigen in patients with clinically amyopathic dermatomyositis: Association with rapidly progressive interstitial lung disease", ARTHRITIS & RHEUMATISM, vol. 60, no. 7, 1 July 2009 (2009-07-01), pages 2193-2200, XP55003397, ISSN: 0004-3591, DOI: 10.1002/art.24621
- SATO S ET AL. ARTHRITIS RHEUM. vol. 52, no. 5, May 2005, pages 1571 - 1576, XP055003396
- MICHITO HIRAKATA JAPANESE JOURNAL OF CHINICAL IMMUNOLOGY vol. 30, no. 6, 2007, pages 444 - 454, XP008144413
- SHINJI SATO MOLECULAR RHEUMATOLOGY vol. 3, no. 1, 2006, pages 3 - 8, XP008144818
- YAMASAKI Y ET AL. ARTHRITIS RHEUM. vol. 56, no. 2, February 2007, pages 596 - 604, XP008144817
- VILLERS A ET AL. ANN DERMATOL VENEREOL. vol. 133, no. 6-7, July 2006, pages 573 - 576, XP008144394
- YOSHIRO HORAI ET AL: "Early diagnosis and treatment for remission of clinically amyopathic dermatomyositis complicated by rapid progress interstitial lung disease: a report of two cases", MODERN RHEUMATOLOGY ; OFFICIAL JOURNAL OF THE JAPAN COLLEGE OF RHEUMATOLOGY, SPRINGER-VERLAG, TO, vol. 23, no. 1, 30 March 2012 (2012-03-30) , pages 190-194, XP035162400, Spinger Inc Tokyo ISSN: 1439-7609, DOI: 10.1007/S10165-012-0637-6
- M. J. FRITZLER ET AL: "Molecular characterization of two human autoantigens: unique cDNAs encoding 95- and 160-kD proteins of a putative family in the Golgi complex", JOURNAL OF EXPERIMENTAL MEDICINE, vol. 178, no. 1, 1 July 1993 (1993-07-01), pages 49-62, XP55176177, Rockefeller University Press, New York NY USA ISSN: 0022-1007, DOI: 10.1084/jem.178.1.49
- H. KATO ET AL: "Differential roles of MDA5 and RIG-I helicases in the recognition of RNA viruses", NATURE, vol. 441, no. 7089, 9 April 2006 (2006-04-09), pages 101-105, XP055094652, Basingstoke UK ISSN: 0028-0836, DOI: 10.1038/nature04734
- S. MOGHADAM-KIA ET AL.: "Anti-Melanoma Differentiation-Associated Gene 5 Is Associated With Rapidly Progressive Lung Disease and Poor Survival in US Patients With Amyopathic and Myopathic Dermatomyositis", ARTHRITIS CARE & RESEARCH, vol. 68, no. 5, 1 May 2016 (2016-05-01), pages 689-694, Hoboken NJ USA
- D-TEK: "MDA-5", 1 October 2015 (2015-10-01), D-TEK, Mons BE, XP55289407, pages 1-2,
- MOISES LABRADOR-HORILLO ET AL: "Anti-MDA5 Antibodies in a Large Mediterranean Population of Adults with Dermatomyositis", JOURNAL OF IMMUNOLOGY RESEARCH, vol. 2014, ID 29079, 4 February 2014 (2014-02-04), pages 1-8, XP55289406, Cairo

### SEQUENCE LISTING

<110> KEIO UNIVERSITY
<120> The diagnostic product for dermatomyositis
<130> P09-69
<150> JP 2008-223789
   <151> 2008-9-1
<160> 4
<170> PatentIn version 3.4
<210> 1
   <211> 1706
   <212> DNA
   <213> Homo sapiens
<400> 1
   gaagaacaca ttttaaaact atgtgccaat cttgatgcat ttactattaa aactgttaaa     60
   gaaaaccttg atcaactgaa aaaccaaata caggagccat gcaagaagtt tgccattgca    120
   gatgcaacca gagaagatcc atttaaagag aaacttctag aaataatgac aaggattcaa    180
   acttattgtc aaatgagtcc aatgtcagat tttggaactc aaccctatga acaatgggcc    240
   attcaaatgg aaaaaaaagc tgcaaaagaa ggaaatcgca aagaacgtgt ttgtgcagaa    300
   catttgagga agtacaatga ggccctacaa attaatgaca caattcgaat gatagatgcg    360
   tatactcatc ttgaaacttt ctataatgaa gagaaagata agaagtttgc agtcatagaa    420
   gatgatagtg atgagggtgg tgatgatgag tattgtgatg gtgatgaaga tgaggatgat    480
   ttaaagaaac ctttgaaact ggatgaaaca gatagatttc tcatgacttt attttttgaa    540
   aacaataaaa tgttgaaaag gctggctgaa aacccagaat atgaaaatga aaagctgacc    600
   aaattaagaa ataccataat ggagcaatat actaggactg aggaatcagc acgaggaata    660
   atctttacaa aaacacgaca gagtgcatat gcgctttccc agtggattac tgaaaatgaa    720
   aaatttgctg aagtaggagt caaagcccac catctgattg gagctggaca cagcagtgag    780
   ttcaaaccca tgacacagaa tgaacaaaaa gaagtcatta gtaaatttcg cactggaaaa    840
   ataaatctgc ttatcgctac cacagtggca gaagaaggtc tggatattaa agaatgtaac    900
   attgttatcc gttatggtct cgtcaccaat gaaatagcca tggtccaggc ccgtggtcga    960
   gccagagctg atgagagcac ctacgtcctg gttgctcaca gtggttcagg agttatcgaa   1020
   catgagacag ttaatgattt ccgagagaag atgatgtata aagctataca ttgtgttcaa   1080
   aatatgaaac cagaggagta tgctcataag attttggaat tacagatgca aagtataatg   1140
   gaaaagaaaa tgaaaaccaa gagaaatatt gccaagcatt acaagaataa cccatcacta   1200
   ataactttcc tttgcaaaaa ctgcagtgtg ctagcctgtt ctggggaaga tatccatgta   1260
   attgagaaaa tgcatcacgt caatatgacc ccagaattca aggaacttta cattgtaaga   1320
   gaaaacaaag cactgcaaaa gaagtgtgcc gactatcaaa taaatggtga aatcatctgc   1380
   aaatgtggcc aggcttgggg aacaatgatg gtgcacaaag gcttagattt gccttgtctc   1440
   aaaataagga attttgtagt ggttttcaaa aataattcaa caaagaaaca atacaaaaag   1500
   tgggtagaat tacctatcac atttcccaat cttgactatt cagaatgctg tttatttagt   1560
   gatgaggatt agcacttgat tgaagattct tttaaaatac tatcagttaa acatttaata   1620
   tgattatgat taatgtattc attatgctac agaactgaca taagaatcaa taaaatgatt   1680
   gttttactct gcaaaaaaaa aaaaaa                                        1706
<210> 2
   <211> 523
   <212> PRT
   <213> Homo sapiens
<400> 2
   Glu Glu His Ile Leu Lys Leu Cys Ala Asn Leu Asp Ala Phe Thr Ile
   1               5                   10                  15
   Lys Thr Val Lys Glu Asn Leu Asp Gln Leu Lys Asn Gln Ile Gln Glu
               20                  25                  30
   Pro Cys Lys Lys Phe Ala Ile Ala Asp Ala Thr Arg Glu Asp Pro Phe
           35                  40                  45
   Lys Glu Lys Leu Leu Glu Ile Met Thr Arg Ile Gln Thr Tyr Cys Gln
       50                  55                  60
   Met Ser Pro Met Ser Asp Phe Gly Thr Gln Pro Tyr Glu Gln Trp Ala
   65                  70                  75                  80
   Ile Gln Met Glu Lys Lys Ala Ala Lys Glu Gly Asn Arg Lys Glu Arg
                   85                  90                  95
   Val Cys Ala Glu His Leu Arg Lys Tyr Asn Glu Ala Leu Gln Ile Asn
               100                 105                 110
   Asp Thr Ile Arg Met Ile Asp Ala Tyr Thr His Leu Glu Thr Phe Tyr
           115                 120                 125
   Asn Glu Glu Lys Asp Lys Lys Phe Ala Val Ile Glu Asp Asp Ser Asp
       130                 135                 140
   Glu Gly Gly Asp Asp Glu Tyr Cys Asp Gly Asp Glu Asp Glu Asp Asp
   145                 150                 155                 160
   Leu Lys Lys Pro Leu Lys Leu Asp Glu Thr Asp Arg Phe Leu Met Thr
                   165                 170                 175
   Leu Phe Phe Glu Asn Asn Lys Met Leu Lys Arg Leu Ala Glu Asn Pro
               180                 185                 190
   Glu Tyr Glu Asn Glu Lys Leu Thr Lys Leu Arg Asn Thr Ile Met Glu
           195                 200                 205
   Gln Tyr Thr Arg Thr Glu Glu Ser Ala Arg Gly Ile Ile Phe Thr Lys
       210                 215                 220
   Thr Arg Gln Ser Ala Tyr Ala Leu Ser Gln Trp Ile Thr Glu Asn Glu
   225                 230                 235                 240
   Lys Phe Ala Glu Val Gly Val Lys Ala His His Leu Ile Gly Ala Gly
                   245                 250                 255
   His Ser Ser Glu Phe Lys Pro Met Thr Gln Asn Glu Gln Lys Glu Val
               260                 265                 270
   Ile Ser Lys Phe Arg Thr Gly Lys Ile Asn Leu Leu Ile Ala Thr Thr
           275                 280                 285
   Val Ala Glu Glu Gly Leu Asp Ile Lys Glu Cys Asn Ile Val Ile Arg
       290                 295                 300
   Tyr Gly Leu Val Thr Asn Glu Ile Ala Met Val Gln Ala Arg Gly Arg
   305                 310                 315                 320
   Ala Arg Ala Asp Glu Ser Thr Tyr Val Leu Val Ala His Ser Gly Ser
                   325                 330                 335
   Gly Val Ile Glu His Glu Thr Val Asn Asp Phe Arg Glu Lys Met Met
               340                 345                 350
   Tyr Lys Ala Ile His Cys Val Gln Asn Met Lys Pro Glu Glu Tyr Ala
           355                 360                 365
   His Lys Ile Leu Glu Leu Gln Met Gln Ser Ile Met Glu Lys Lys Met
       370                 375                 380
   Lys Thr Lys Arg Asn Ile Ala Lys His Tyr Lys Asn Asn Pro Ser Leu
   385                 390                 395                 400
   Ile Thr Phe Leu Cys Lys Asn Cys Ser Val Leu Ala Cys Ser Gly Glu
                   405                 410                 415
   Asp Ile His Val Ile Glu Lys Met His His Val Asn Met Thr Pro Glu
               420                 425                 430
   Phe Lys Glu Leu Tyr Ile Val Arg Glu Asn Lys Ala Leu Gln Lys Lys
           435                 440                 445
   Cys Ala Asp Tyr Gln Ile Asn Gly Glu Ile Ile Cys Lys Cys Gly Gln
       450                 455                 460
   Ala Trp Gly Thr Met Met Val His Lys Gly Leu Asp Leu Pro Cys Leu
   465                 470                 475                 480
   Lys Ile Arg Asn Phe Val Val Val Phe Lys Asn Asn Ser Thr Lys Lys
                   485                 490                 495
   Gln Tyr Lys Lys Trp Val Glu Leu Pro Ile Thr Phe Pro Asn Leu Asp
               500                 505                 510
   Tyr Ser Glu Cys Cys Leu Phe Ser Asp Glu Asp
           515                 520
<210> 3
   <211> 3434
   <212> DNA
   <213> Homo sapiens
<400> 3
   gccccgctgc ccacctgccc gcctgcccac ctgcccaggt gcgagtgcag ccccgcgcgc     60
   cggcctgaga gccctgtgga caacctcgtc attgtcaggc acagagcggt agaccctgct    120
   tctctaagtg ggcagcggac agcggcacgc acatttcacc tgtcccgcag acaacagcac    180
   catctgcttg ggagaaccct ctcccttctc tgagaaagaa agatgtcgaa tgggtattcc    240
   acagacgaga atttccgcta tctcatctcg tgcttcaggg ccagggtgaa aatgtacatc    300
   caggtggagc ctgtgctgga ctacctgacc tttctgcctg cagaggtgaa ggagcagatt    360
   cagaggacag tcgccacctc cgggaacatg caggcagttg aactgctgct gagcaccttg    420
   gagaagggag tctggcacct tggttggact cgggaattcg tggaggccct ccggagaacc    480
   ggcagccctc tggccgcccg ctacatgaac cctgagctca cggacttgcc ctctccatcg    540
   tttgagaacg ctcatgatga atatctccaa ctgctgaacc tccttcagcc cactctggtg    600
   gacaagcttc tagttagaga cgtcttggat aagtgcatgg aggaggaact gttgacaatt    660
   gaagacagaa accggattgc tgctgcagaa aacaatggaa atgaatcagg tgtaagagag    720
   ctactaaaaa ggattgtgca gaaagaaaac tggttctctg catttctgaa tgttcttcgt    780
   caaacaggaa acaatgaact tgtccaagag ttaacaggct ctgattgctc agaaagcaat    840
   gcagagattg agaatttatc acaagttgat ggtcctcaag tggaagagca acttctttca    900
   accacagttc agccaaatct ggagaaggag gtctggggca tggagaataa ctcatcagaa    960
   tcatcttttg cagattcttc tgtagtttca gaatcagaca caagtttggc agaaggaagt   1020
   gtcagctgct tagatgaaag tcttggacat aacagcaaca tgggcagtga ttcaggcacc   1080
   atgggaagtg attcagatga agagaatgtg gcagcaagag catccccgga gccagaactc   1140
   cagctcaggc cttaccaaat ggaagttgcc cagccagcct tggaagggaa gaatatcatc   1200
   atctgcctcc ctacagggag tggaaaaacc agagtggctg tttacattgc caaggatcac   1260
   ttagacaaga agaaaaaagc atctgagcct ggaaaagtta tagttcttgt caataaggta   1320
   ctgctagttg aacagctctt ccgcaaggag ttccaaccat ttttgaagaa atggtatcgt   1380
   gttattggat taagtggtga tacccaactg aaaatatcat ttccagaagt tgtcaagtcc   1440
   tgtgatatta ttatcagtac agctcaaatc cttgaaaact ccctcttaaa cttggaaaat   1500
   ggagaagatg ctggtgttca attgtcagac ttttccctca ttatcattga tgaatgtcat   1560
   cacaccaaca aagaagcagt gtataataac atcatgaggc attatttgat gcagaagttg   1620
   aaaaacaata gactcaagaa agaaaacaaa ccagtgattc cccttcctca gatactggga   1680
   ctaacagctt cacctggtgt tggaggggcc acgaagcaag ccaaagctga agaacacatt   1740
   ttaaaactat gtgccaatct tgatgcattt actattaaaa ctgttaaaga aaaccttgat   1800
   caactgaaaa accaaataca ggagccatgc aagaagtttg ccattgcaga tgcaaccaga   1860
   gaagatccat ttaaagagaa acttctagaa ataatgacaa ggattcaaac ttattgtcaa   1920
   atgagtccaa tgtcagattt tggaactcaa ccctatgaac aatgggccat tcaaatggaa   1980
   aaaaaagctg caaaagaagg aaatcgcaaa gaacgtgttt gtgcagaaca tttgaggaag   2040
   tacaatgagg ccctacaaat taatgacaca attcgaatga tagatgcgta tactcatctt   2100
   gaaactttct ataatgaaga gaaagataag aagtttgcag tcatagaaga tgatagtgat   2160
   gagggtggtg atgatgagta ttgtgatggt gatgaagatg aggatgattt aaagaaacct   2220
   ttgaaactgg atgaaacaga tagatttctc atgactttat tttttgaaaa caataaaatg   2280
   ttgaaaaggc tggctgaaaa cccagaatat gaaaatgaaa agctgaccaa attaagaaat   2340
   accataatgg agcaatatac taggactgag gaatcagcac gaggaataat ctttacaaaa   2400
   acacgacaga gtgcatatgc gctttcccag tggattactg aaaatgaaaa atttgctgaa   2460
   gtaggagtca aagcccacca tctgattgga gctggacaca gcagtgagtt caaacccatg   2520
   acacagaatg aacaaaaaga agtcattagt aaatttcgca ctggaaaaat aaatctgctt   2580
   atcgctacca cagtggcaga agaaggtctg gatattaaag aatgtaacat tgttatccgt   2640
   tatggtctcg tcaccaatga aatagccatg gtccaggccc gtggtcgagc cagagctgat   2700
   gagagcacct acgtcctggt tgctcacagt ggttcaggag ttatcgaaca tgagacagtt   2760
   aatgatttcc gagagaagat gatgtataaa gctatacatt gtgttcaaaa tatgaaacca   2820
   gaggagtatg ctcataagat tttggaatta cagatgcaaa gtataatgga aaagaaaatg   2880
   aaaaccaaga gaaatattgc caagcattac aagaataacc catcactaat aactttcctt   2940
   tgcaaaaact gcagtgtgct agcctgttct ggggaagata tccatgtaat tgagaaaatg   3000
   catcacgtca atatgacccc agaattcaag gaactttaca ttgtaagaga aaacaaagca   3060
   ctgcaaaaga agtgtgccga ctatcaaata aatggtgaaa tcatctgcaa atgtggccag   3120
   gcttggggaa caatgatggt gcacaaaggc ttagatttgc cttgtctcaa aataaggaat   3180
   tttgtagtgg ttttcaaaaa taattcaaca aagaaacaat acaaaaagtg ggtagaatta   3240
   cctatcacat ttcccaatct tgactattca gaatgctgtt tatttagtga tgaggattag   3300
   cacttgattg aagattcttt taaaatacta tcagttaaac atttaatatg attatgatta   3360
   atgtattcat tatgctacag aactgacata agaatcaata aaatgattgt tttactctgc   3420
   aaaaaaaaaa aaaa                                                     3434
<210> 4
   <211> 1025
   <212> PRT
   <213> Homo sapiens
<400> 4

## Description

The present invention relates to a method and the use of a kit for diagnosing clinically amyopathic dermatomyositis, and use of an antigenic protein for diagnosing clinically amyopathic dermatomyositis.

A basic characteristic of connective tissue disease that causes it to be regarded as an autoimmune disease is the production of autoantibodies against various cellular components. It is known that many of the antigens corresponding to such autoantibodies are enzymes and regulatory factors that are essential to life processes. Investigating the molecular structures of the autoantibodies and antigens corresponding thereto (autoantigens), and their biological functions is expected to contribute to the elucidation of the pathogenesis of connective tissue diseases.

Polymyositis/dermatomyositis (PM/DM) is an inflammatory myopathy in which proximal muscle weakness and muscular pain caused by skeletal muscle inflammation predominate. Particularly, when typical skin symptoms such as heliotrope rash and Gottron's papules are observed, the patient is diagnosed with DM. Polymyositis/dermatomyositis is one of the autoimmune diseases, and various autoantibodies are known to develop. The presence of antibodies in the sera of PM/DM patients has been reported; such antibodies include anti-aminoacyl tRNA synthetase antibody (anti-ARS antibody), anti-SRP antibody, anti-Mi-2 antibody, and like antibodies specifically detected in myositis patients, as well as anti-U1RNP antibody, anti-SS-A antibody, and like autoantibodies associated with myositis. It is known that patients who are positive for the same myositis-specific autoantibody exhibit similar clinical characteristics; this is clinically useful for diagnosis, classification of disease types, selection of appropriate treatment methods, and estimation of prognosis.

In contrast, autoantibody negativity was considered to be one of the characteristics of clinically amyopathic DM (C-ADM), which is a sub-type of PM/DM. The presence of autoantibodies specific to C-ADM was unknown. It was known that C-ADM is resistant to clinical treatment and associated with poor prognostic rapidly progressive interstitial lung disease (RP-ILD). To save the life of C-ADM patients with RP-ILD, the efficacy of potent treatment from an early stage using a combination of high dose steroid therapy and immunosuppressive drug administration has been reported and recommended. For this reason, early diagnosis of C-ADM with associated RP-ILD is clinically important, and the establishment of new indicators that are useful for early diagnosis has been desired.

In light of the above background, the present inventors investigated the sera of normal healthy controls, those of patients with connective tissue diseases including C-ADM, and those of patients with idiopathic pulmonary fibrosis, according to an immunoprecipitation (IPP) method. The inventors found that a new autoantibody capable of recognizing a 140-kDa protein is present in the sera of C-ADM patients, and termed the new autoantibody "anti-CADM-140 antibody" (Non-Patent Literature 1 (NPL 1)).

The anti-CADM-140 antibody was not detected in any of the patients with connective tissue diseases other than C-ADM, patients with idiopathic pulmonary fibrosis (IPF), and normal healthy controls. Clinically, significantly frequent development of RP-ILD in anti-CADM-140 antibody-positive patients was observed, which suggests a correlation between the anti-CADM-140 antibody and RP-ILD (Non-Patent Document 2).

This phenomenon is considered to be useful for early diagnosis and treatment selection for extremely poor prognostic C-ADM with associated RP-ILD, and is thus expected to improve its prognosis.

MDA5 has a SNP (http://www.ncbi.nlm.nih.gov/SNP/snp_ref.cgi?locusId=64135). The amino acid sequence and the base sequence thereof are available under NCBI Accession Number NM_022168. Patent Literature 1 (PTL 1) discloses the relationship between MDA5 and cancer.

The present inventors published Non-Patent Literature 3 (NPL 3), which discloses the closest prior art. Departing from NPL3, the present invention provides methods and usages of kits for the early diagnosis of C-ADM, which are more amenable to large scale applications than the method of NPL3 and which do not require the use of radio-labelled components as does the method of NPL3.

### Citation List

### Patent Literature

PTL 1: Japanese Unexamined Patent Document No. 2003-531581 (Fisher, P.B. et al.; the Trustees of Columbia University in the City of New York)
PTL 2: US-patent 6,160,107 (Targoff, I. N. et al.; Oklahoma Medical Research Foundation) describes nucleic acids encoding antigens associated with polymyositis and dermatomyositis.

### Non-patent Literature

NPL 1: Shinji Sato, Akira Suwa, Takaki Nojima, Misako Suzuki, Yuko Kaneko, Masataka Kuwana, Shinichi Inada, Nasashi Akizuki, Takeji Nishikawa, Michito Hirakata; Clinical and Immunological Features of Autoantibodies of the 140 kDa Polypeptide in Patients with Amyopathic Dermatomyosis; Arthritis Rheum. 46(9): S398, 2002.
NPL 2: Shinji Sato, Michito Hirakata, Masataka Kuwana, Akira Suwa, Shinichi Inada, Tsuneyo Mimori, Takeji Nishikawa, Chester V. Oddis, and Yasuo Ikeda; Autoantibodies to a 140-kd Polypeptide, CADM-140, in Japanese Patients With Clinically Amyopathic Dermatomyositis; Arthritis Rheum. 52(5): 1571-1576, 2005.
NPL 3: Shinji Sato, Kana Hoshino, Takashi Satoh, Tomonobu Fujita, Yutaka Kawakami, Takashi Fujita, and Masataka Kuwana; RNA Helicase Encoded by Melanoma Differentiation-Associated Gene 5 Is a Major Autoantigen in Patients With Clinically Amyopathic Dermatomyositis: Association with Rapidly Progressive Interstitial Lung Disease; Arthritis Rheum. 60(7): 2193-2200, 2009.
NPL 4: Michito Hirakata; Jpn. J. Clin. Immunol., 30(6), 444-454, 2007, describes autoantibodies and their clinical significance in idiopathic inflammatory myopathies; polymyositis/dermatomyositis and related conditions.
NPL 5: Shinji Sato; Molecular Rheumatology, vol. 3, no. 1, 2006, 3-8, describes novel antibodies in polymyositis/dermatomyositis;
NPL 6: Yamasaki Y. et al.; Arthritis Rheum. 56(2): 596-604, 2007, describes autoantibodies to RNA helicase A.
NPL 7: Villers A. et al.; Ann. Dermatol. Venereol., 133(6-7), 573-576, 2006, describes regression of primary melanoma with metastases associated with amyopathic dermatomyositis.

Further, document Sato (Sato, S.; Molecular Rheumatology, 3(1); 2006; pp. 3-8) describes novel antibodies in the diagnosis of polymyositis and dermatomyositis. Document Fritzler *et al*. (Fritzler, M. J. et al.; Journal of Experimental Medicine, 178(1); 1993; pp. 49-62) describes the molecular characterization of human autoantigens encoded by unique cDNAs encoding 95 and 160 kDa proteins of a putative family in the Golgi complex. Kato et *al.* (Kato, H. et al.; Nature, 441(7089); 2006; pp. 101-105) describes differential roles of MDA5 and RIG-I helicases in the recognition of RNA viruses. Further, WO 2005/016962 A2 describes protein-containing compositions and methods of using the same for the diagnosis and treatment of immune related diseases such as idiopathic inflammatory myopathies. Furthermore, Horai et al.; Modern Rheumatology, 23(1); 2012; pp. 190-194) describes two cases of an early diagnosis and treatment for remission of clinically amyopathic dermatomyositis complicated by rapid progress interstitial lung disease. Moreover, document "D-tek: MDA5" describes a kit comprising MDA5 for the detection of antibodies directed against the same. Finally, Labrador-Horillo *et al.* (Labrador-Horillo, M. et al.; Journal of Immunology Research; 2014; pp. 1-8) describes anti-MDA5 antibodies in a large mediterranean population of adults with dermatomyositis.

In NPL3 anti -CADM-140 antibody has been generally measured according to an IPP method using S³⁵-labeled leukemia-derived K562 cell extracts or HeLa cells. Although this is a reliable measurement method with high sensitivity and high specificity, the method utilizes isotopes and requires complicated procedures. Accordingly, the measurement method has been used only in a limited number of laboratories.

To apply anti-CADM-140 antibody measurement to actual clinical diagnosis, it is necessary to establish a measurement system that enables easy measurement of large quantities of samples. Identification of an antigen corresponding to anti-CADM-140 antibody, preparation of a recombinant protein, and establishment of a measurement system by ELISA etc., are important to establish such a system.

The present inventors used a HeLa cell cDNA library to clone an antigen gene corresponding to anti-CADM-140 antibody, and investigated the molecular sequence of the corresponding antigen protein. As a result, the inventors found that MDA5 (Melanoma Differentiation Associated Gene 5) is an antigen corresponding to anti-CADM-140 antibody and have accomplished the present invention based on this finding.

The present invention provides uses of kits and methods for diagnosing clinically amyopathic dermatomyositis according to the appended claims.

According to the present invention, extremely poor prognostic clinically amyopathic dermatomyositis associated with a high risk for developing extremely poor prognostic rapidly progressive interstitial lung disease can be detected with high accuracy.

The present invention enables quick measurement of anti-CADM-140 antibody in many samples. This is useful for early diagnosis and treatment selection for C-ADM with associated RP-ILD, and is expected to improve the prognosis of this disease whose treatment method has not been established and which has been considered as an extremely poor prognostic disease. Furthermore, the accumulation of anti-CADM-140 antibody-positive samples will enable the analysis of foreign antigens, such as a virus that is the target of a specific autoantibody, and contribute to the elucidation of the pathogenesis of C-ADM with associated RP-ILD.

The figures show:

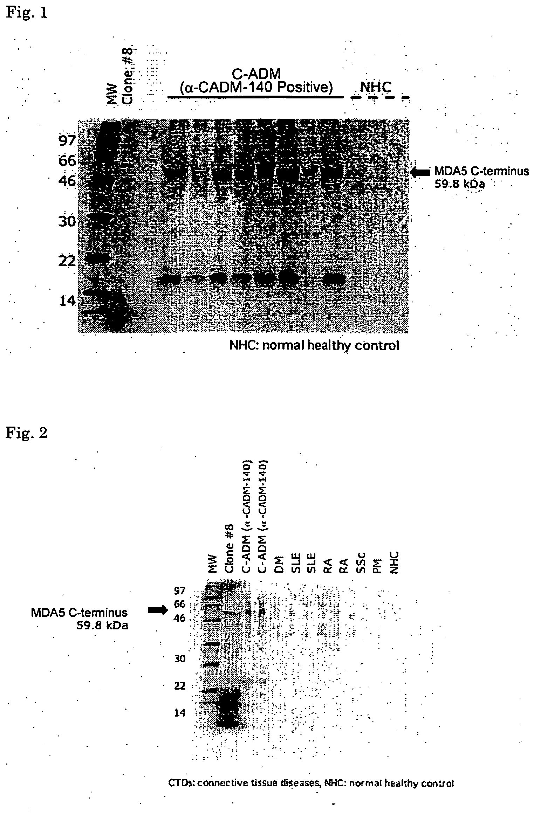

[FIG. 1] FIG. 1 shows the results of investigation of a reaction between a protein of Clone #8 (MDA5) as an antigen and the sera of C-ADM patients according to an immunoprecipitation method, which was performed after purifying the protein of Clone #8 (MDA5) and confirming the expression of the protein using an in vitro transcription/translation system. The Clone #8 (MDA5) reacted with all of the anti-CADM-140 antibody-positive serum samples, but it did not react with the sera of normal healthy controls.
[FIG. 2] FIG. 2 shows the results of investigation of a reaction between a recombinant protein of Clone #8 (MDA5) as an antigen, and the sera of anti-CADM-140 antibody-positive C-ADM patients, those of patients with other connective tissue diseases, and those of normal healthy controls, according to an immunoprecipitation method, which was performed after purifying the protein of Clone #8 (MDA5) and confirming expression of the protein using an in vitro transcription/translation system. The recombinant protein did not react with the sera of normal healthy controls or the sera of patients other than anti-CADM-140 antibody-positive C-ADM patients.

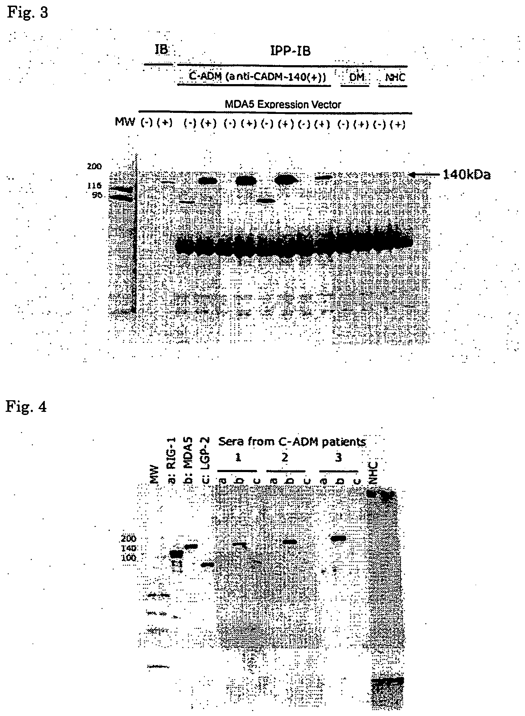

[FIG. 3] FIG. 3 shows the IPP-IB results. When anti-CADM-140 antibody was reacted with an MDA5-expressing African green monkey renal cell-derived COS7 cell extract, the resulting 140-kDa immunoprecipitate reacted with goat anti-MDA5 antibody.
[FIG. 4] FIG. 4 shows the immunoblotting results of the sera of anti-CADM-140 antibody-positive patients and those of normal healthy controls, obtained by using recombinant RIG-I, recombinant MDA5, and recombinant LGP-2 as antigens. The sera of anti-CADM-140 antibody-positive patients reacted only with the recombinant MDA5, whereas the sera of normal healthy controls (NHC) did not react at all.

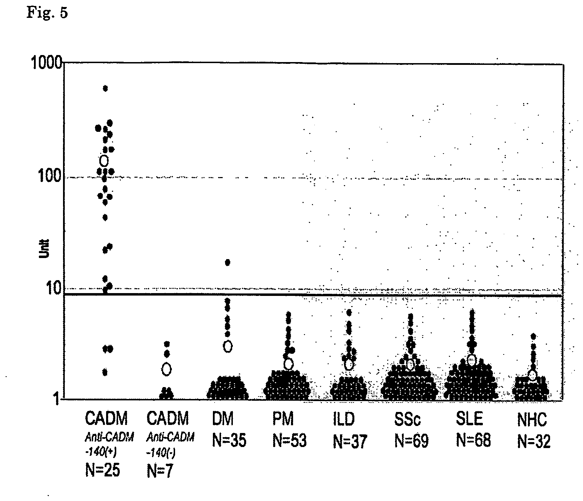

[FIG. 5] FIG. 5 shows the ELISA results of the sera of anti-CADM-140 antibody-positive C-ADM patients, those of anti-CADM-140 antibody-negative C-ADM patients, those of PM/DM patients, those of scleroderma (SSc) patients, those of systemic lupus erythematosus (SLE) patients, those of ILD patients, and those of normal healthy controls, obtained by using MDA5 as an antigen substrate. The cutoff value is indicated by a horizontal line. The analytical sensitivity and analytical specificity of this ELISA system were 85% and 100%, respectively.

A feature of the present invention is an antigen (MDA5) specifically recognized by anti-CADM-140 antibody, which was found by the present inventor. MDA5 is one type of RIG-I family protein. RIG-I family proteins participate in a specific immune response that includes interferon type I production. RIG-I family proteins are highly homologous and contain a DexD/H-box helicase domain. In addition to MDA5, RIG-I and LPG-2 are also known as RIG-I family proteins. These proteins are very similar in sequence and structure, and are thus likely to be cross-reactive. Accordingly, the reactivity between anti-CADM-140 antibody and RIG-I family proteins was also investigated. The binding of anti-CADM-140 antibody to MDA5 depends on the conformation of the antigen, and anti-CADM-140 antibody preferentially reacts with an MDA5 partially modified from its native structure. These factors made it difficult to determine that anti-CADM-140 antibody is an antibody to MDA5.

To identify anti-CADM-140 antibody, the present inventors purified an antigen protein corresponding thereto and tried to identify the antigen protein by mass spectrometry. However, it was impossible to purify a sufficient amount of the antigen protein, and identification was difficult. This was considered to be attributable to low amounts of antigen in the serum or to the affinity of the antigen-antibody reaction.

Further, in the early stages of C-ADM, it is difficult to distinguish the disease from other types of dermatomyositis, because C-ADM is difficult to diagnose and requires follow-up to carefully determine whether muscular symptoms are present or not.

However, the present inventors identified MDA5 specifically recognized by anti-CADM-140 antibody. As a result, it became clear that the clinical sensitivity and clinical specificity of the ELISA assay of dermatomyositis (C-ADM) are 69% and 99.6%, respectively. Although anti-CADM-140 antibody-positive C-ADM patients were previously difficult to diagnose or the diagnosis often took much time, the present invention enables quick diagnosis.

In this specification, "dermatomyositis" includes clinically amyopathic dermatomyositis (C-ADM), which is a subtype of DM, but does not include polymyositis PM. Thus, the present invention provides a kit and a method for diagnosing whether the subject has C-ADM or other diseases. DM is an inflammatory myopathy in which characteristic skin symptoms such as heliotrope rash and Gottron's papules, and proximal muscle weakness and/or muscular pain caused by skeletal muscle inflammation predominate. DM is generally diagnosed according to the diagnostic criteria of Bohan & Peter (Bohan A, Peter JB. Polymyositis and dermatomyositis. N Engl J Med 1975; 292: 344). However, C-ADM, which refers to the case in which cutaneous lesions characteristic of DM are exhibited but muscle weakness is not exhibited, does not meet the criteria of Bohan & Peter, and has been undiagnosable. In 2002, Sontheimer proposed new classification criteria in which a case of typical skin lesions without muscle weakness is called "C-ADM" (clinically amyopathic dermatomyositis), and "C-ADM" is classified as a subtype of DM [Sontheimer RD: Would a new name hasten the acceptance of amyopathic dermatomyositis (dermatomyositis sine myositis) as a distinctive subset within the idiopathic inflammatory dermatomyopathies spectrum of clinical illness? J Am Acad Dermatol 2002; 46: 626-36]. The target DM to be diagnosed according to the present invention includes those that meet the classification criteria of Sontheimer.

The diagnostic kit used in the present invention and the diagnostic method of the present invention use an MDA5 protein or a fragment thereof recognized by anti-CADM-140 antibody to detect anti-CADM-140 antibody (hereinafter the "fragment recognized by anti-CADM-140 antibody" is sometimes simply referred to as an "immunogenic peptide"). Since the C-terminal 523-amino-acid sequence fragment (SEQ ID NO: 2) of MDA5 protein shown in SEQ ID NO: 4 binds to anti-CADM-140 antibody, an epitope recognized by anti-CADM-140 antibody is present in the C-terminal region of MDA5 protein. Insofar as this epitope is maintained, the MDA5 protein or the immunogenic peptide thereof generally may be a modified MDA5 protein or immunogenic peptide thereof that has one or more amino acid deletions, substitutions, additions, or insertions in a non-epitopic region thereof. The epitope typically consists of about 5 to about 10 amino acids, and particularly about 5 to about 8 amino acids. For example, the epitope of MDA5 can be determined by synthesizing a specific number of peptides (for example, 10 peptides at a time) while shifting by a fixed number of amino acids (for example, 5 amino acids) from the N-terminus of the C-terminal fragment of MDA5 shown in SEQ ID NO: 2 (for example, prepare a peptide consisting of the first to tenth amino acids, a peptide consisting of the sixth to 15th amino acids, a peptide consisting of the 11th to 20th amino acids, a peptide consisting of the 16th to 25th amino acids, ...), and then investigating the reactivity of each of the peptide fragments with anti-CADM-140 antibody. When the epitope is determined or the location of the epitope is narrowed down, the MDA5 protein (immunogenic peptide) fragment recognized by anti-CADM-140 antibody can be shortened. Generally, a shorter immunogenic peptide is preferable from the viewpoint of ease of production. A peptide consisting of 40 or less amino acids, 30 or less amino acids, or 20 or less amino acids, particularly about 5 to about 10 amino acids, is preferable. RNA helicase is encoded by MDA5.

MDA5 is known to have an SNP. SEQ ID NO: 3 represents a base sequence of MDA5. SEQ ID NO: 1 represents a base sequence encoding a C-terminal fragment of the MDA5 fragment shown in SEQ ID NO: 2. However, the MDA5 of other amino acid sequences/base sequences that are related by SNP, or alleles of MDA5 are also included in the scope of "MDA5" in this specification.

The MDA5 or the immunogenic peptide used in the present invention is expressed as a recombinant protein and produced by genetic engineering, or is produced by chemical synthesis. Generally, when the immunogenic peptide has a short sequence consisting of preferably 50 or less amino acids, more preferably 30 or less, even more preferably 20 or less, and particularly, 5 to 10 amino acids, chemical synthesis (a solid or liquid phase synthesis) is preferably used to produce the peptide. The MDA5 or the immunogenic peptide thereof may be used in the form of a monomer, and may be crosslinked by a polyfunctional crosslinking agent such as glutaraldehyde, or can be produced by genetic engineering by using DNA encoding a peptide in which immunogenic peptides are connected in tandem.

The anti-CADM-140 antibody may be obtained from any sample such as the blood, serum, plasma, cerebrospinal fluid, and lymph of a subject. Blood, serum, and plasma are preferable, and serum is particularly preferable as the sample.

The kit used in diagnosing dermatomyositis according to the present invention contains an MDA5 protein or an immunogenic peptide thereof. The kit may contain a substance for detecting an antigen-antibody complex of an MDA5 protein or an immunogenic peptide thereof, and anti-CADM-140 antibody. Examples of such substances include anti-human immunoglobulin antibodies that are labeled with peroxidases such as horseradish peroxidase (HRP), enzyme labels such as β-galactosidase, alkaline phosphatase, and luciferase, fluorescent labels such as FITC (fluorescein isothiocyanate) and RITC (tetramethylrhodamin isothiocyanate), or any other suitable labels, and particularly anti-human IgG antibodies labeled therewith.

Immunoassay is preferably used for the diagnostic kit used in the present invention and the diagnostic method of the present invention. Examples of the immunoassay include enzyme immunoassay (EIA), ELISA, fluoroimmunoassay (FIA), chemiluminescent immunoassay, immunoblotting (IB), Western blotting, immunostaining, and like methods.

The MDA5 protein or the immunogenic peptide used in the present invention is preferably bonded to a solid phase. Examples of such solid phases include agarose, wells of a microtiter plate, latex particles, and the like. Specific examples of the ELISA method include competitive immunoassay, sandwich immunoassay, and the like.

According to the method for diagnosing dermatomyositis of the present invention, a sample obtained from a subject is brought into contact with an MDA5 protein shown in SEQ ID NO: 4 or an immunogenic peptide thereof to detect the presence or absence of an antigen-antibody complex of MDA5 or an immunogenic peptide thereof and anti-CADM-140 antibody by a physical or chemical method.

The method for diagnosing dermatomyositis of the present invention comprises the steps as defined in item 5. above.

The diagnostic kit used in the present invention may contain:
(i) an antigen comprising an MDA5 protein shown in SEQ ID NO: 4 or an immunogenic peptide thereof (the antigen may be adhered to wells of a microtiter plate etc., and may further be blocked with a blocking agent such as skim milk, or an albumin such as bovine serum albumin (BSA) or I) ;
(ii) a medium suitable for the reaction between a sample of a subject and the antigen (i) (for example, buffers such as PBS);
(iii) a reagent for detecting a complex of the antigen (i) and anti-CADM-140 antibody (for example, a labeled anti-human immunoglobulin antibody that can bind to anti-CADM-140 antibody); and optionally
(iv) a reference sample not containing anti-CADM-140 antibody.

The "reference sample not containing anti-CADM-140 antibody" includes, for example, blood, serum, and plasma samples of normal healthy controls.

When the anti-human immunoglobulin antibody is labeled with peroxidase, anti-CADM-140 antibody can be detected by a process comprising adding a color development substrate such as tetramethylbenzidine; stopping the reaction with H₂SO₄; and measuring absorbance (450 nm: OD450) using a plate reader.

By testing many samples using the diagnostic kit according to the present invention and comparing the obtained results with other clinical observations, more accurate criteria for determining whether a subject has clinically amyopathic DM, based on the measured amount of anti-CADM-140 antibody can be established.

### Examples

The present invention is described below in more detail.

### Example 1: Method for early diagnosis of C-ADM

An antigenic protein was expressed in E. coli by using an E. coli (XL1-Blue MRF) expressing lambda ZAP phage prepared by using a HeLa cell cDNA library. The expressed protein was transferred to a nitrocellulose membrane, and reacted with anti-CADM-140 antibody-positive serum to isolate positive clones. Next, the reactivity of the obtained positive clones with 10 serum samples of anti-CADM-140 antibody-positive C-ADM patients, 14 serum samples of anti-CADM-140 antibody-negative persons (two C-ADM samples, two PM samples, one sample each of systemic lupus erythematosus, scleroderma, and interstitial lung disease, and seven samples of normal healthy controls) was investigated. Among the 9 clones that were obtained, Clone #8 reacted with anti-CADM-140 antibody-positive serum samples at high frequency, i.e. 9 out of 10 anti-CADM-140 antibody-positive C-ADM serum samples. After the protein was purified and expression was confirmed using an in vitro transcription/translation assay, the reactivity of the sera of C-ADM patients with the protein as an antigen was investigated by immunoprecipitation. Clone #8 reacted with all of the anti-CADM-140 antibody-positive serum samples, but did not react with the sera of normal healthy controls (FIG. 1). Further, Clone #8 did not react with the sera of patients with connective tissue diseases other than anti-CADM-140 antibody-positive C-ADM (FIG. 2). Further, to determine the base sequence of Clone #8, plasmid DNA was excised from phage DNA from which the clone was obtained. The plasmid DNA was purified to determine the base sequence. Finally, a homology search was performed for the obtained base sequence, and the sequence was perfectly matched to the C-terminal sequence of MDA5.

An anti-CADM-140 antibody-positive serum was reacted with an MDA5-expressing African green monkey renal cell-derived COS7 cell extract by the IPP-IB method. The reactivity of 140 kDa of the obtained immunoprecipitate with goat anti-MDA5 antibody was investigated. When MDA5 was expressed, the immunoprecipitate reacted with goat anti-MDAS antibody (FIG. 3). Further, the reactivity of anti-CADM-140 antibody-positive sera and the sera of normal healthy controls with recombinant purified proteins of RIG-I family RIG-I, MD5, and LGP-2 as antigens was investigated. The sera of anti-CADM-140 antibody-positive patients reacted only with recombinant MDA5, whereas the sera of normal healthy controls did not react with recombinant MDA5 (FIG. 4). It became clear from the above results that the antigen corresponding to anti-CADM-140 antibody is MDA5.

Further, the present inventors coated a 96-well plate with purified recombinant MDA5 as an antigen to establish an ELISA for an anti-ACDM-140 antibody assay. The diagnostic sensitivity and diagnostic specificity of the anti-CADM-140 antibody assay method were investigated.

More specifically, recombinant MDA5 was immobilized on a 96-well ELISA plate in an amount of 0.05 µg/well and blocked with 3% BSA. Serum samples (each 250-fold diluted) were dispensed to the plate in an amount of 100 µl per well, and allowed to react at room temperature for 2 hours. As a secondary antibody, 5000-fold diluted peroxidase conjugated goat anti-human-IgG was used. Tetramethylbenzidine (1 mg/ml) was added as a color development substrate, and the reaction was stopped with H₂SO₄. The absorbance was measured with a plate reader (450 nm: OD₄₅₀). Using this assay system, the serum samples of C-ADM-containing collagen disease patients, those of interstitial lung disease (ILD) patients, and those of normal healthy controls were measured. The results showed that the reactivity of the sera of the anti-CADM-140 antibody-positive PM/DM patients was higher than that of the anti-CADM-140 antibody-negative PM/DM patients, scleroderma (SSc) patients, systemic lupus erythematosus (SLE) patients, ILD patients, and normal healthy controls (FIG. 5). When the average + up to 10 times the standard derivation of normal healthy controls was defined as the normal cutoff range, the analytical sensitivity and the analytical specificity measured by this ELISA assay system were 85% and 100%, respectively. Thus, the results clearly show that this assay is an anti-CADM-140 antibody detection method with excellent sensitivity and specificity.

## Claims

1. Use of a kit for diagnosing clinically amyopathic dermatomyositis (C-ADM), the kit comprising the recombinant or chemically synthesized Melanoma Differentiation Associated Gene 5 (MDA5) protein consisting of SEQ ID NO: 4, or an MDA5 specific immunogenic peptide of said MDA5 protein, wherein said MDA5 specific immunogenic peptide of said MDA5 protein is a C-terminal fragment of the MDA5 protein, said fragment consisting of SEQ ID NO: 2.

2. The use according to claim 1 of a kit according to claim 1 for diagnosing C-ADM associated with a high risk for developing rapidly progressive interstitial lung disease.

3. The use according to claim 1 of a kit according to claim 1 for diagnosing C-ADM, wherein the kit is used for measurement by enzyme-linked immunosorbent assay (ELISA);
wherein said MDA5 protein or said MDA5 specific immunogenic peptide of said MDA5 protein is bound to a solid phase.

4. A method for diagnosing c-ADM, comprising allowing a sample of a subject to react with the recombinant or chemically synthesized MDA5 protein consisting of SEQ ID NO: 4, or an MDA5 specific immunogenic peptide of said MDA5 protein; and diagnosing the subject as having C-ADM when an antibody against said MDA5 protein or said MDA5 specific immunogenic peptide of said MDA5 protein consisting of SEQ ID NO: 2 is detected in the sample;
wherein the sample is a blood, serum, or plasma sample.

5. The method according to claim 4 wherein the antibody against said MDA5 protein or said MDA5 specific immunogenic peptide of said MDA5 protein in the sample is detected by ELISA, and wherein the method comprises the following steps:
(i) depositing a specific amount of the recombinant or chemically synthesized MDA5 protein consisting of SEQ ID NO: 4, or an MDA5 specific immunogenic peptide of said MDA5 protein consisting of SEQ ID NO: 2 on a plurality of wells of a microtiter plate;
(ii) diluting a sample of a subject suspected of having C-ADM, and adding the diluted sample to the wells;
(iii) washing the microtiter plate after incubation;
(iv) introducing a labeled anti-human immunoglobulin antibody to the wells of the microtiter plate; and
(v) detecting the amount of label bound to the antibody by comparison with a control.

6. Use of the recombinant or chemically synthesized Melanoma Differentiation Associated Gene 5 (MDA5) protein consisting of SEQ ID NO: 4, or an MDA5 specific immunogenic peptide of said MDA5 protein consisting of ID SEQ NO: 2 to diagnose clinically amyopathic dermatomyositis (C-ADM).

## Patentansprüche

1. Verwendung eines Kits für die Diagnose klinisch amyopathischer Dermatomyositis (C-ADM), wobei der Kit das rekombinant oder chemisch synthetisierte *Melanoma Differentiation Associated Gene* 5 (MDA5)-Protein, bestehend aus SEQ ID NO: 4, oder ein MDA5-spezifisches immunogenes Peptid des MDA5-Proteins umfaßt, wobei das MDA5-spezifische immunogene Peptid des MDA5-Proteins ein C-terminales Fragment des MDA5-Proteins ist, wobei das Fragment aus SEQ ID NO: 2 besteht.

2. Verwendung nach Anspruch 1 eines Kits nach Anspruch 1 für die Diagnose von C-ADM, welche mit einem hohen Risiko für die Entwicklung von rasch fortschreitender interstitieller Lungenerkrankung verbunden ist.

3. Verwendung nach Anspruch 1 eines Kits nach Anspruch 1 für die Diagnose von C-ADM, wobei der Kit für eine Messung mittels *enzyme-linked immunosorbent assay* (ELISA) verwendet wird,
wobei das MDA5-Protein oder das MDA5-spezifische immunogene Peptid des MDA5-Proteins an eine feste Phase gebunden ist.

4. Verfahren zur Diagnose von c-ADM, umfassend das Reagierenlassen einer Probe eines Subjekts mit dem rekombinant oder chemisch synthetisierten MDA5-Protein, bestehend aus SEQ ID NO: 4, oder einem MDA5-spezifischen immunogenen Peptid des MDA5-Proteins, und Diagnostizieren des Subjekts als C-ADM aufweisend, wenn ein Antikörper gegen das MDA5-Protein oder das MDA5-spezifische immunogene Peptid des MDA5-Proteins, bestehend aus SEQ ID NO: 2, in der Probe nachgewiesen wird,
wobei die Probe eine Blut-, Serum- oder Plasmaprobe ist.

5. Verfahren nach Anspruch 4, wobei der Antikörper gegen das MDA5-Protein oder das MDA5-spezifische immunogene Peptid des MDA5-Proteins in der Probe mittels ELISA nachgewiesen wird und wobei das Verfahren die folgenden Schritte umfasst:
(i) Ablegen einer spezifischen Menge des rekombinant oder synthetisch synthetisierten MDA5-Proteins, bestehend aus SEQ ID NO: 4, oder eines MDA5-spezifischen immunogenen Peptids des MDA5-Proteins, bestehend aus SEQ ID NO: 2, in einer Vielzahl von Vertiefungen einer Mikrotiterplatte,
(ii) Verdünnen einer Probe eines Subjekts, das im Verdacht steht, C-ADM aufzuweisen, und Zugeben der verdünnten Probe zu den Vertiefungen,
(iii) Waschen der Mikrotiterplatte nach Inkubation,
(iv) Einbringen eines markierten Antikörpers, der gegen humanes Immunglobulin gerichtet ist, in die Vertiefungen der Mikrotiterplatte und
(v) Nachweisen der Menge der an den Antikörper gebunden Markierung durch Vergleich mit einer Kontrolle.

6. Verwendung des rekombinant oder chemisch synthetisierten *Melanoma Differentiation Associated Gene 5* (MDA5)-Proteins, bestehend aus SEQ ID NO: 4, oder eines MDA5-spezifischen immunogenen Peptids des MDA5-Proteins, bestehend aus SEQ ID NO: 2, für die Diagnose klinisch amyopathischer Dermatomyositis (C-ADM).

## Revendications

1. Utilisation d'un kit pour le diagnostic de la dermatomyosite cliniquement amyopathique (C-ADM), le kit comprenant la protéine du gène 5 associé à la différenciation des mélanomes (MDA5) recombinante ou synthétisée chimiquement constituée de SEQ ID NO : 4, ou un peptide immunogène spécifique de MDA5 de ladite protéine MDA5 dans lequel ledit peptide immunogène spécifique de MDA5 de ladite protéine MDA5 est un fragment C-terminal de la protéine MDA5, ledit fragment étant constitué de SEQ ID NO : 2.

2. Utilisation selon la revendication 1 d'un kit selon la revendication 1 pour le diagnostic de la C-ADM associée à un risque élevé de développer rapidement une maladie pulmonaire interstitielle progressive.

3. Utilisation selon la revendication 1 d'un kit selon la revendication 1 pour le diagnostic de la C-ADM, dans laquelle le kit est utilisé pour une mesure par la technique ELISA (enzyme-linked immunosorbent assay) ;
dans laquelle ladite protéine MDA5 ou ledit peptide immunogène spécifique de MDA5 de ladite protéine MDA5 est lié à une phase solide.

4. Procédé de diagnostic de la C-ADM, comprenant l'étape permettant à un échantillon d'un sujet de réagir avec la protéine MDA5 recombinante ou synthétisée chimiquement constituée de SEQ ID NO : 4, ou un peptide immunogène spécifique de MDA5 de ladite protéine MDA5 ; et le diagnostic du sujet comme souffrant de C-ADM lorsqu'un anticorps dirigé contre ladite protéine MDA5 ou ledit peptide immunogène spécifique de MDA5 de ladite protéine MDA5 constitué de SEQ ID NO : 2 est détecté dans l'échantillon ;
dans lequel l'échantillon est un échantillon de sang, de sérum, ou de plasma.

5. Procédé selon la revendication 4 dans lequel l'anticorps dirigé contre ladite protéine MDA5 ou ledit peptide immunogène spécifique de MDA5 de ladite protéine MDA5 dans l'échantillon est détecté par la technique ELISA, et dans lequel le procédé comprend les étapes suivantes :
(i) le dépôt d'une quantité spécifique de la protéine MDA5 recombinante ou synthétisée chimiquement constituée de SEQ ID NO : 4, ou d'un peptide immunogène spécifique de MDA5 de ladite protéine MDA5 constitué de SEQ ID NO : 2 dans une pluralité de puits d'une plaque de microtitrage ;
(ii) la dilution d'un échantillon d'un sujet suspecté de souffrir de C-ADM, et l'addition de l'échantillon dilué aux puits ;
(iii) le lavage de la plaque de microtitrage après incubation ;
(iv) l'introduction d'un anticorps anti-immunoglobuline humaine marqué dans les puits de la plaque de microtitrage ; et
(v) la détection de la quantité de marqueur lié à l'anticorps par comparaison à un témoin.

6. Utilisation de la protéine du gène 5 associé à la différenciation des mélanomes (MDA5) recombinante ou synthétisée chimiquement constituée de SEQ ID NO : 4, ou d'un peptide immunogène spécifique de MDA5 de ladite protéine MDA5 constitué de SEQ ID NO : 2 pour diagnostiquer la dermatomyosite cliniquement amyopathique (C-ADM).
